# EUROPÄISCHE PATENTSCHRIFT

(11) **EP 0 451 519 B1**
(45) Veröffentlichungstag und Bekanntmachung des Hinweises auf die Patenterteilung: **14.12.1994**
(21) Anmeldenummer: 91103786.9
(22) Anmeldetag: 13.03.1991
(51) Int. Cl.: C07D 207/404

(54) **Verfahren zur Herstellung von N-Succinimidylcarbonaten**
Process for the preparation of N-succinimidylcarbonates
Procédé pour la préparation de carbonates de N-succinimidyle

(30) Priorität: 10.04.1990 AT 839/90
(43) Veröffentlichungstag der Anmeldung: 16.10.1991
(73) Patentinhaber: Chemie Linz GmbH, A-4021 Linz (AT)
(72) Erfinder: Steinbauer, Gerhard, Dipl.-Ing. Dr., A-4470 Enns (AT); Huber, Wolfgang, Mag.Dr., A-5020 Salzburg (AT); Kögler, Rupert, A-4020 Linz (AT)
(74) Vertreter: Kunz, Ekkehard, Dr.

(56) Entgegenhaltungen:
- US-A- 4 038 282
- US-A- 4 126 628
- CHEMICAL ABSTRACTS, Band 66, Nr, 1, 2. Januar 1967, Columbus Ohio, USA JONES J.H. et al. "Preparation of p-methoxybenzyloxycarbonylamino acids" page 315, columns 1,2, abstract-No. 2 749q & Chem. Ind. (London), 41 1722 (1966)

## Beschreibung

Die Erfindung betrifft ein Verfahren zur Herstellung von N-Succinimidylcarbonaten durch Phosgenierung von Alkoholen zu den entsprechenden Chlorkohlensäureestern und anschließende Umsetzung mit N-Hydroxysuccinimid.

N-Succinimidylcarbonate werden in der Synthese von Peptiden zur Einführung geeigneter Schutzgruppen für die Aminofunktion von Aminosäuren verwendet. Die N-Succinimidylcarbonate werden aufgrund ihrer selektiveren Reaktion gegenüber den entsprechenden Säurechloriden bevorzugt. Eine besonders gut geeignete Schutzgruppe ist die 9-Fluorenylmethyloxycarbonylgruppe, da diese sehr leicht unter milden basischen Bedingungen abgespalten werden kann.

Zur Herstellung von N-Succinimidylcarbonaten sind im wesentlichen zwei Reaktionswege bekannt.

In Ten Kortenaar et al., Int. Peptide Protein Research 27 (1986), 398ff ist die Herstellung von 9-Fluorenylmethylsuccinimidylcarbonat durch Phosgenierung von 9-Fluorenylmethanol und anschließende Umsetzung mit N-Hydroxysuccinimid beschrieben. Die Phosgenierung erfolgt durch Zudosieren des Alkohols in flüssiges Phosgen bei einer Temperatur von -78°C. Der entstandene Chlorkohlensäureester wird isoliert, in Dioxan gelöst und mit N-Hydroxysuccinimid in Gegenwart von Triethylamin als Neutralisationsmittel bei einer Temperatur von 10 - 15°C umgesetzt.

Nach L.A. Carpino, G.Y-Han, J. Org. Chem. Vol 37, (1972) 3404 ff. kann die Phosgenierung auch durch Zugabe des Alkohols zu einer Lösung von Phosgen in Dichlormethan durchgeführt werden.

Bei beiden Verfahren ist in der Lösung eine sehr hohe Konzentration an Phosgen vorhanden, was aus sicherheitstechnischen Gründen bedenklich ist. Das überschüssige Phosgen muß nach beendeter Phosgenierung entfernt werden. Der bei der Phosgenierung als Nebenprodukt entstandene Chlorwasserstoff würde bei der anschließenden Umsetzung neben dem Chlorkohlensäureester mit dem als Neutralisationsmittel eingesetzten organischen Amin zum entsprechenden Aminhydrochlorid reagieren. Er muß daher ebenfalls entfernt werden. Die entstandenen Aminhydrochloride sind außerdem umweltbedenkliche Nebenprodukte, die entsorgt werden müssen. Ferner entsteht bei der Umsetzung des Chlorkohlensäureester mit N-Hydroxysuccinimid in Gegenwart eines organischen Amins aufgrund der basischen Labilität des Fluorenylesters leicht das Spaltprodukt Dibenzofulven, wodurch die Ausbeute vermindert wird.

Nach A. Paquet, Can. J. Chem 60 (1982), 976ff kann die Herstellung von 9-Fluorenylmethylsuccinimidylcarbonat auch durch Phosgenierung des Cyclohexylaminsalzes von N-Hydroxysuccinimid und anschließende Umsetzung des Succinimidylchlorkohlensäureesters mit 9-Fluorenylmethanol erfolgen. Dabei wird zu einer Suspension des N-Hydroxysuccinimidsalzes bei -30°C flüssiges Phosgen eingeleitet, das entstandene Dicyclohexylaminhydrochlorid wird abgetrennt und der Succinylimidylchlorkohlensäureester isoliert. Die anschließende Umsetzung mit 9-Fluorenylmethanol erfolgt in Dichlormethan in Gegenwart von Pyridin als Neutralisationsmittel.

Auch bei diesen Verfahren müssen vor der Umsetzung des Chlorkohlensäureesteresters, die bei der Phosgenierung entstandenen Nebenprodukte, sowie das überschüssige Phosgen entfernt werden. Es entsteht sowohl bei der Phosgenierung als auch bei der weiteren Umsetzung des Chlorkohlensäureesters ein Aminhydrochlorid, das entsorgt werden muß.

Für eine Herstellung von N-Hydroxysuccinimidylcarbonaten im technischen Maßstab sind die bekannten Verfahren demnach schlecht geeignet.

Es konnte nun ein Verfahren zur Herstellung von N-Succinimidylcarbonaten gefunden werden, bei dem das Zwischenprodukt nicht isoliert werden muß und die entstandenen anorganischen Salze leicht abgetrennt werden können.

Gegenstand der Erfindung ist demnach ein Verfahren zur Herstellung von N-Succinimidylcarbonaten der Formel I 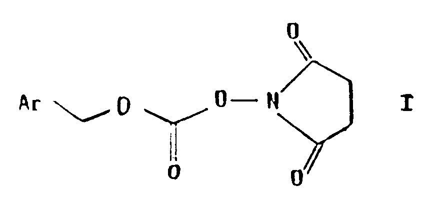
in der Ar einen 9-Fluorenylrest, einen Phenylrest, der gegebenenfalls durch Halogen, Nitro, Alkyl mit 1 - 4 C-Atomen oder Trifluormethyl substituiert ist, oder einen 5- oder 6-gliedrigen heteroaromatischen Rest mit ein oder zwei N- oder S-Atomen bedeutet, das dadurch gekennzeichnet ist, daß man einen Alkohol der Formel II,

Ar - CH₂ OH

in der Ar die oben angegebene Bedeutung hat mit Phosgen in Gegenwart eines inerten mit Wasser mischbaren Verdünnungsmittel zu einem Chlorkohlensäureester der Formel III, 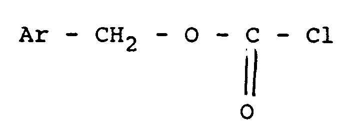
reagieren läßt und die Reaktionsmischung ohne Isolierung der Verbindung der Formel III mit einer wäßrigen Lösung von N-Hydroxysuccinimid in Gegenwart eines Alkali-, Erdalkali- oder Ammoniumhydrogencarbonats oder -carbonats als Neutralisationsmittel umsetzt und die Verbindung der Formel I aus der organischen Phase isoliert.

Als Ausgangsverbindungen werden Alkohole der Formel II verwendet, in der Ar einen Fluorenylrest oder einen Phenylrest bedeutet, der gegebenenfalls ein- oder mehrfach durch Halogen, beispielsweise Chlor, Brom oder Fluor , Nitro, Alkyl mit 1 - 4 C-Atomen oder Trifluormethyl substituiert sein kann. Beispiele für solche substituierten Phenylreste sind der 2-Chlorphenylrest, der 4-Chlorphenylrest, der 2,4-Dichlorphenylrest, der 2-Bromphenylrest, der 4-Fluorphenylrest, der Methylphenylrest, der Trifluormethylphenylrest. Ferner kann Ar einen 5- oder 6-gliedrigen heteroaromatischen Ring bedeuten, der als Heteroatome einm oder zwei N-oder S-Atome enthält, beispielsweise einen Pyridyl-, einen Pyridazinyl-, einen Pyrimidyl- oder einen Thienylrest.

Vorzugsweise werden solche Alkohole der Formel II eingesetzt, in der Ar den 9-Fluorenyl-, den Phenyl- oder den 2-Chlorphenylrest bedeutet.

Die Umsetzung des Alkohols mit Phosgen erfolgt in einem unter Reaktionsbedingungen inerten mit Wasser mischbaren Verdünnungsmittel, beispielsweise in einem cyclischen Ether wie Tetrahyrofuran oder Dioxan oder in einem offenkettigen Ether wie einem Diether des Di-, Tri- oder Tetraethylenglykol. Die Reaktionspartner werden üblicherweise in äquivalenten Mengen eingesetzt. Zur Vervollständigung der Reaktion kann es vorteilhaft sein, einen etwa 10 %igen Überschuß an Phosgen einzusetzen.

Bei der Durchführung der Reaktion ist es vorteilhaft den Alkohol im Verdünnungsmittel vorzulegen und Phosgen einzuleiten, da dadurch die Konzentration an Phosgen in der Lösung gering gehalten wird. Die Phosgenierungsreaktion erfolgt bei Temperaturen von etwa 0 - 20°C.

Die aus der Phosgenierung erhaltene Reaktionslösung wird zu einer wäßrigen Lösung gegebenenfalls in Mischung mit dem inerten Verdünnungsmittel von N-Hydroxysuccinimid und einem Alkali-, Erdalkali- oder Ammoniumhydrogencarbonat oder -carbonat zudosiert.

Pro Mol Alkohol wird üblicherweise 1 Mol N-Hydroxysuccinimid eingesetzt. Bei der Reaktion entstehen pro Mol Phosgen 2 Mol HCl. Zur Neutralisation sind daher 2 Äquivalente Neutralisationsmittel erforderlich. Beispiele für geeignete Neutralisationsmittel sind Kaliumhydrogencarbonat, Kaliumcarbonat, Natriumhydrogencarbonat, Natriumcarbonat, Ammoniumhydrogencarbonat oder Ammoniumcarbonat.

Chlorkohlensäureester sind, je nach Stabilität der Alkoholkomponente, mehr oder weniger hydrolyseempfindlich. Bei den anmeldungsgemäß verwendeten Chlorkohlensäureestern findet jedoch beim Einbringen in die wäßrige Lösung keine Hydrolyse des Chlorkohlensäureesters und anschließende Decarboxylierung zum Alkohol statt, es findet eine praktisch vollständige Umsetzung zum N-Succinimidylcarbonat statt. Das aus der Phosgenierung gegebenenfalls vorhandene überschüssige Phosgen wird sofort hydrolysiert. Als Nebenprodukt fallen die entsprechenden Alkali-, Erdalkali- oder Ammoniumchloride und gegebenenfalls CO₂ an.

Gegen Ende der Umsetzung findet eine Phasentrennung statt, wobei die wäßrige Phase das Alkali-, Erdalkali- oder Ammoniumchlorid enthält.
Die organische Phase enthält das N-Succinimidylcarbonat, das leicht durch Abdampfen des Lösungsmittels isoliert werden kann.

### Beispiel 1:

98,1 g (0,50 mol) Fluoren-9-methanol wurden in 300 ml Tetrahydrofuran gelöst und die Lösung auf 15°C abgekühlt.
Innerhalb von 30 Minuten wurden unter Kühlung 54,4 g (0,55 mol) Phosgen eingeleitet. Anschließend wurde die Lösung 1,5 Stunden bei 15°C nachgerührt (1. Stufe). In einem zweiten Reaktionsgefäß wurden 63,3 g (0,55 mol)N-Hydroxysuccinimid 115,1 g (1,15 mol) Kaliumhyrogencarbonat, 350 ml Wasser und 300 ml Tetrahyrofuran vorgelegt. Die Reaktionslösung aus der 1. Stufe wurde rasch dosiert und anschließend eine Stunde gerührt.
Gegen Ende der Reaktion fand eine Phasentrennung statt.
Die Tetrahydrofuranphase wurde abgetrennt und eingedampft.

Es wurden 162 g 9-Fluorenylmethylsuccinimidylcarbonat erhalten. Die Ausbeute betrug 96 % der Theorie bezogen auf eingesetztes Fluoren-9-methanol. Eine Analyse durch HPLC ergab einen Gehalt von 98 %. Durch Digerieren mit Isopropanol kann ein Produkt in 99,9 % Reinheit erhalten werden.
¹H-NMR (CDCl3): delta = 7,77 (d. J = 7,5 Hz; 2H) 7,62 (d. J= 7,5 Hz; 2H), 7,43 (t, J= 7,5 HZ, 2H), 7,36 (t, J= 7,5 Hz; 2H), 4,56 (d. J= 7,5 Hz; 2H), 4,33 (t, J= 7,5 Hz; 1H), 2,80 (s; 4H) ppm.

### Beispiel 2:

54,1 g (0,50 mol) Benzylalkohol wurden in 300 ml Tetrahydrofuran gelöst. In diese Lösung wurden unter Kühlung innerhalb einer Stunde 49,9 g (0,505 mol) Phosgen eingeleitet und 30 Minuten bei 15°C nachgerührt (1. Stufe).
In ein weiteres Reaktionsgefäß wurde eine Lösung von 57,5 g (0,5 mol) N-Hydroxysuccinimid, 105,1 g Kaliumhyrogencarbonat (1,05 mol), 350 ml Wasser und 300 ml Tetrahydrofuran vorgelegt. Die Reaktionslösung aus der 1. Stufe wurde rasch dosiert und 20 Minuten gerührt. Gegen Ende der Reaktion fand eine Phasentrennung statt. Die Tetrahydrofuranphase wurde abgtrennt und eingedampft.
Es wurden 120 g Benzylsuccinimidylcarbonat erhalten. Die Ausbeute betrug 96 % der Theorie, bezogen auf den eingesetzten Benzylalkohol.
¹H-NMR (CDCl₃): delta = 7,40 (s;5H), 5,,32 (s; 2H), 2,82 (s; 4H) ppm.

### Beispiel 3:

71,3 g (0,50 mol) 2-Chlorbenzylalkohol wurden in 300 ml Tetrahydrofuran gelöst. In diese Lösung wurde unter Kühlung innerhalb von 45 Minuten 54,4 g Phosgen (0,550 mol) eingeleitet und 1 Stunde bei 15°C nachgerührt (1. Stufe).

In ein weiteres Reaktionsgefäß wurde eine Lösung von 57,5 g (0,5 mol) N-Hydroxysuccinimid, 115,1 g Kaliumhydrogencarbonat (1,150 mol), 350 ml Wasser und 300 ml Tetrahydrofuran vorgelegt.
Die Reaktionslösung aus der 1. Stufe wurde rasch dosiert und eine Stunde gerührt. Gegen Ende der Reaktion fand eine Phasentrennung statt. Die Tetrahydrofuranphase wurde abgetrennt und eingedampft.
Es wurden 138 g 2-Chlorbenzylsuccinimidylcarbonat erhalten. Die Ausbeute betrug 97 % der Theorie, bezogen auf 2-Chlorbenzylalkohol.
¹H-NMR (CDCl₃): delta = 7,3 - 7,5 (m; 4H), 5,43 (s; 2H), 2,80 (s; 4H) ppm.

### Beispiel 4:

44,3 g (0,25 mol) 2,4-Dichlorbenzylalkohol wurden in 300 ml Tetrahydrofuran gelöst. In diese Lösung wurde unter Kühlung innerhalb von 45 Minuten 27,2 g Phosgen (0,272 mol) eingeleitet und 1 Stunde bei 15°C nachgerührt (1. Stufe).

In ein weiteres Reaktionsgefäß wurde eine Lösung von 28,8 g (0,25 mol) N-Hydroxysuccinimid, 57,5 g Kaliumhydrogencarbonat (0,575 mol), 350 ml Wasser und 300 ml Tetrahydrofuran vorgelegt. Die Reaktionslösung aus der 1. Stufe wurde rasch dosiert und eine Stunde gerührt. Gegen Ende der Reaktion fand eine Phasentrennung statt. Die Tetrahydrofuranphase wurde abgetrennt und eingedampft.
Es wurden 75 g 2,4-Dichlorbenzylsuccinimidylcarbonat erhalten. Die Ausbeute betrug 94 % der Theorie, bezogen auf 2,4-Dichlorbenzylalkohol.
¹H-NMR (CDCl₃): delta = 7,27 - 7,45 (m; 3H), 5,40 (s; 2H), 2,84 (s;4H) ppm.

### Beispiel 5:

76,6 g (0,50 mol) 4-Nitrobenzylalkohol wurden in 300 ml Tetrahydrofuran gelöst. In diese Lösung wurde unter Kühlung innerhalb von 45 Minuten 54,4 g Phosgen (0,550 mol) eingeleitet und 1 Stunde bei 15°C nachgerührt. (1. Stufe).

In ein weiteres Reaktionsgefäß wurde eine Lösung von 57,5 g (0,5 mol) N-Hydroxysuccinimid, 115,1 g Kaliumhydrogencarbonat (1,150 mol), 350 ml Wasser und 300 ml Tetrahydrofuran vorgelegt.
Die Reaktionslösung aus der 1. Stufe wurde rasch dosiert und eine Stunde gerührt. Gegen Ende der Reaktion fand eine Phasentrennung statt. Die Tetrahydrofuranphase wurde abgetrennt und eingedampft.
Es wurden 140 g, 4-Nitrobenzylsuccinimidylcarbonat erhalten. Die Ausbeute betrug 95 % der Theorie, bezogen auf 4-Nitrobenzylalkohol.
¹H-NMR (CDCl₃): delta = 8,27 (d. J. = 8,7 Hz; 2H) 7,58 (d. J. = 8,7 Hz; 2H), 5,42 (s, 1H), 2,86 (s,4H) ppm.

### Beispiel 6:

80,1 g (0,40 mol) 3-Phenoxybenzylalkohol wurden in 300 ml Tetrahydrofuran gelöst. In diese Lösung wurde unter Kühlung innerhalb von 45 Minuten 43,5 g Phosgen (0,440 mol) eingeleitet und 1 Stunde bei 15°C nachgerührt (1. Stufe).

In ein weiteres Reaktionsgefäß wurde eine Lösung von 46.0 g (0,4 mol) N-Hydroxysuccinimid, 92,1 g Kaliumhydrogencarbonat (0,92 mol), 350 ml Wasser und 300 ml Tetrahydrofuran vorgelegt. Die Reaktionslösung aus der 1. Stufe wurde rasch dosiert und eine Stunde gerührt. Gegen Ende der Reaktionszeit fand eine Phasentrennung statt. Die Tetrahydrofuranphase wurde abgetrennt und eingedampft.
Es wurden 127 g 3-Phenoxybenzylsuccinimidylcarbonat erhalten. Die Ausbeute betrug 93 % der Theorie, bezogen auf 3-Phenoxybenzylalkohol.
¹H-NMR (CDCl₃): delta = 7,01 - 8,38 (m; 10H), 5,27 (s; 2H), 2,83 (s; 4H) ppm.

## Patentansprüche

1. Verfahren zur Herstellung von N-Succinimidylcarbonaten der Formel 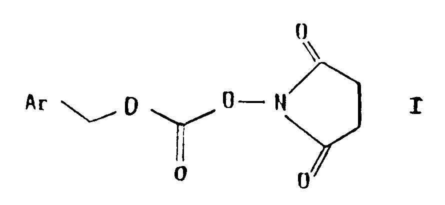 in der Ar einen 9-Fluorenylrest, einen Phenylrest, der gegebenenfalls durch Halogen, Nitro, Alkyl mit 1 - 4 C-Atomen oder Trifluormethyl substituiert ist, oder einen 5- oder 6-gliedrigen heteroaromatischen Rest mit ein oder zwei N- oder S-Atomen bedeutet, dadurch gekennzeichnet, daß man einen Alkohol der Formel
Ar - CH₂ OH II
in der Ar die oben angegebenen Bedeutung hat mit Phosgen in Gegenwart eines inerten mit Wasser mischbaren Verdünnungsmittels zu einem Chlorkohlensäureester der Formel 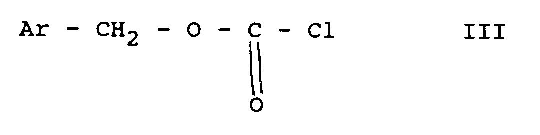 reagieren läßt und die Reaktionsmischung ohne Isolierung der Verbindung der Formel III mit einer wäßrigen Lösung von N-Hydroxysuccinimid in Gegenwart eines Alkali-, Erdalkali- oder Ammoniumhydrogencarbonats oder -carbonats als Neutralisationsmittel umsetzt und die Verbindung der Formel I aus der organischen Phase isoliert.

2. Verfahren nach Anspruch 1, dadurch gekennzeichnet, daß als inertes Verdünnungsmittel ein cyclischer oder offenkettiger Ether verwendet wird.

3. Verfahren nach einem der Ansprüche 1 oder 2, dadurch gekennzeichnet, daß als Neutralisationsmittel Natriumhydrogencarbonat, Natriumcarbonat, Kaliumhydrogencarbonat, Kaliumcarbonat, Ammoniumhydrogencarbonat oder Ammoniumcarbonat verwendet wird.

4. Verfahren nach einem der Ansprüche 1 bis 3, dadurch gekennzeichnet, daß ein Alkohol der Formel II eingesetzt wird, in der Ar den 9-Fluorenylrest, den Phenylrest oder den 2-Chlorphenylrest bedeutet.

## Claims

1. Process for preparing N-succinimidyl carbonates of the formula 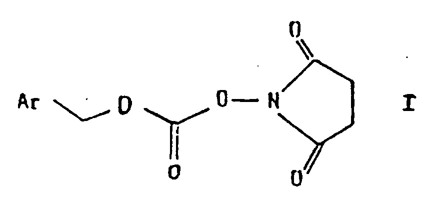 in which Ar denotes a 9-fluorenyl radical, a phenyl radical, which is optionally substituted by halogen, nitro, alkyl having 1-4 C atoms or trifluoromethyl, or a 5- or 6-membered heteroaromatic radical having one or two N or S atoms, characterized in that an alcohol of the formula
Ar - CH₂ OH
in which Ar has the abovementioned meaning, is reacted with phosgene in the presence of an inert diluent, which is miscible with water, to form a chlorocarbonic ester of the formula 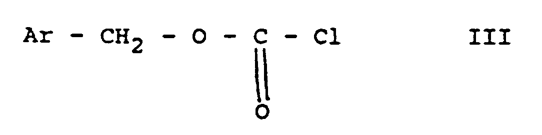 and the reaction mixture, without isolation of the compound of the formula III, is reacted with an aqueous solution of N-hydroxysuccinimide, in the presence of an alkali metal hydrogen carbonate or carbonate, alkaline earth metal hydrogen carbonate or carbonate, or ammonium hydrogen carbonate or carbonate, as neutralizing agent, and the compound of the formula I is isolated from the organic phase.

2. Process according to Claim 1, characterized in that a cyclic or open-chain ether is used as inert diluent.

3. Process according to either of Claims 1 or 2, characterized in that sodium hydrogen carbonate, sodium carbonate, potassium hydrogen carbonate, potassium carbonate, ammonium hydrogen carbonate or ammonium carbonate is used as neutralizing agent.

4. Process according to any of Claims 1 to 3, characterized in that an alcohol of the formula II in which Ar denotes the 9-fluorenyl radical, the phenyl radical or the 2-chlorophenyl radical is employed.

## Revendications

1. Procédé de préparation de carbonates de N-succinimidyle de formule 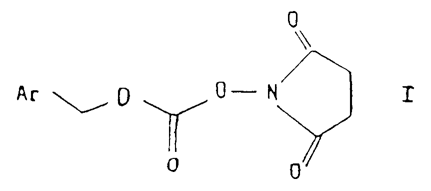 dans laquelle Ar signifie un reste 9-fluorényle, un reste phényle qui est éventuellement substitué par un halogène ou par un groupe nitro ou alkyle en C₁₋₄ ou trifluorométhyle, ou Ar signifie un reste hétéro-aromatique à 5 ou 6 chaînons avec un ou deux atomes d'azote ou de soufre, caractérisé en ce qu'on fait réagir un alcool de formule :
Ar - CH₂OH II
dans laquelle Ar a la signification indiquée ci-dessus, avec un phosgène en présence d'un diluant inerte miscible avec l'eau, pour obtenir un chlorocarbonate de formule : 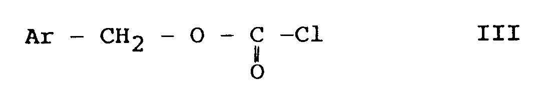 et on fait réagir le mélange de réaction, sans isoler le composé de formule III, avec une solution aqueuse de N-hydroxysuccinimide en présence d'un hydrogénocarbonate ou d'un carbonate alcalin, alcalino-terreux ou d'ammonium, comme agent de neutralisation, et on isole le composé de formule I de la phase organique.

2. Procédé selon la revendication 1, caractérisé en ce qu'on utilise, comme diluant inerte, un éther cyclique ou un éther à chaîne ouverte.

3. Procédé selon l'une des revendications 1 et 2, caractérisé en ce qu'on utilise, comme agent de neutralisation, l'hydrogénocarbonate de sodium, le carbonate de sodium, l'hydrogénocarbonate de potassium, le carbonate de potassium, l'hydrogénocarbonate d'ammonium ou le carbonate d'ammonium.

4. Procédé selon l'une des revendications 1 à 3, caractérisé en ce qu'on utilise un alcool de formule II où Ar signifie le reste 9-fluorényle, le reste phényle ou le reste 2-chlorophényle.
